# EUROPEAN PATENT SPECIFICATION

(11) **EP 1 680 075 B1**
(45) Date of publication and mention of the grant of the patent: **02.01.2008**
(21) Application number: 04790338.0
(22) Date of filing: 11.10.2004
(51) Int. Cl.: A61K 8/26, A61K 8/31, A61K 8/34, A61K 8/41, A61K 8/90, A61Q 5/12

(54) **HAIR CONDITIONING COMPOSITIONS**
HAARKONDITIONIERUNGSZUSAMMENSETZUNGEN
COMPOSITIONS APRES-SHAMPOOING

(30) Priority: 21.10.2003 EP 03256626
(43) Date of publication of application: 19.07.2006
(73) Proprietor: UNILEVER PLC, London EC4P 4BQ (GB); UNILEVER N.V., 3013 AL Rotterdam (NL)
(72) Inventor: GILES, Colin, C. D. Unilever Thai Holdings Ltd, Lakyao, Jatuka, 10900 Bangkok (TH)
(74) Representative: Newbould, Frazer Anthony
(86) International application number: PCT/EP2004/011463
(87) International publication number: WO 2005/039517

(56) References cited:
- EP-A- 1 266 652
- WO-A-02/19976
- WO-A-99/25312

## Description

This invention relates to a hair conditioning composition, to a method of treating hair using the composition and to the use of certain materials for imparting conditioning benefits to hair.

Hair conditioning compositions have been available for several years. The invention relates to rinse-off hair conditioning compositions which are typically intended for use after the hair has been washed and can impart properties to the hair such as smoothness, shine, ease of combing, improved style retention and improved hold. The compositions are generally applied to clean hair whilst it is still wet after having been shampooed, and are then rinsed off prior to drying the hair.

Different consumers desire differing properties for wet and dry hair, therefore, there remains a need for compositions that can impart improved conditioning benefits to hair and/or an altered profile of the properties that make up what are perceived by the user of the compositions as hair conditioning benefits. For example, there can be an advantage in increasing the perceived smoothness of hair which can be provided by a conditioning composition compared to other hair conditioning properties.

Certain hydrophobically modified clays have been incorporated into hair treatment compositions as suspending or anti-agglomerating agents.

For example, US 4,983,383 discloses hair care compositions which are said to give both improved style and hair conditioning properties. Hydrophobically modified clays are described as being suitable suspending/antiagglomerating agents for silicone gums in hairspray forms of the compositions disclosed. The hydrophobically modified clays are, accordingly, employed in the examples of hairspray compositions that are given in the document. However, there is no suggestion of using these materials in compositions other than hairsprays or of any other benefit that the clays might possess.

US 5,846,549 discloses cosmetic compositions intended for cleaning the hair, the scalp and/or the skin. The compositions employ the clay as an agent for dispersing an insoluble silicone in the composition. Generally, the clay is not modified. The compositions are cleaning compositions, such as shampoos, and there is no mention of hair conditioning compositions.

JP-A-05/246,824 discloses hair cosmetics which are water-in-oil emulsions. The cosmetics contain an organically modified clay material, a quaternary ammonium salt, a water-soluble high molecular weight substance and a silicone. The cosmetics are stated as being non-sticky and as imparting lustre, smooth feel and firmness.

JP-A-61/066,752 describes a gel composition containing a silicone oil, a polyoxyethylene-modified silicone and an organically modified montmorillonite clay. The compositions aim to have good stability.

US 5,679,327 describes highly alkaline hair straightening emulsions. Examples cited in the disclosure include a modified hectorite clay gellant with cationic surfactant and high levels of cetearyl alcohol.

WO 99/25312 discloses fluidized polymer suspensions. An example in the disclosure contains Tixogel MP 100 clay as a suspending agent along with cationic surfactant and cetyl alcohol. The weight ratio of cationic surfactant to fatty alcohol is 1:0.67.

A copending application, WO 2004/000250, discloses hair conditioning compositions comprising water, a cationic surfactant, a hydrophobically modified clay and a fatty alcohol in combination with hair modifying agents that are capable of reducing the stiffness of hair fibre. The compositions disclosed give improved smoothness and slipperiness for dry hair, giving the perception to the consumer of moisturisation of the hair.

It has now been found that this effect can be enhanced by the incorporation of certain low HLB surfactants into the compositions.

### Summary of the invention

According to a first aspect of the invention, there is provided A hair conditioning composition with a pH of 8 or less comprising:
(a) 0.01% to 10% by weight of one or more cationic surfactants;
(b) 0.01% to 10% by weight of one or more fatty alcohols having from 8 to 22 carbon atoms;
(c) 0.001% to 5% by weight of a hydrophobically modified clay; and
(d) from 10% to 95% by weight of water, and
(e) from 0.001% to 10% by weight of a hair fibre modifying agent that is capable of reducing the stiffness of a hair fibre,
wherein the weight ratio of cationic surfactant to fatty alcohol is from 1:1 to 1:10, characterised in that the composition further comprises from 0.01 to 5% by weight of a block copolymer according to the formula 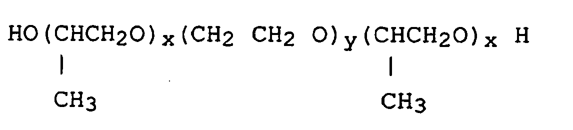 wherein the mean value of y is from 2 to 30 and the mean value x is from 12 to 30 and further the ratio x/y is from 1 to 6.

A second aspect of the invention is a method of treating the hair with such a composition.

In another aspect, the invention is concerned with the use of such compositions to give improved smoothness and slipperiness for dried hair.

### Detailed description of the invention

The hair conditioning compositions of the invention are rinse off compositions ie, they are intended to be rinsed from the hair (typically with water) after use. Such rinse off compositions are distinguished from leave on compositions that are intended not to be rinsed off the hair but instead left on the hair, until after the hair is dry.

The hair conditioning compositions of the invention are typically aqueous-based compositions which comprise an aqueous phase, optionally comprising a gelled phase and/or an insoluble oil phase dispersed and/or suspended in the aqueous phase. As such, the compositions are not water-in-oil emulsions.

Compositions according to the invention have a pH of 8 or less, preferably 7 or less, more preferably 6.5 or less. Suitably, the pH is 2 or more, preferably 2.5 or more or more.

### Hydrophobically Modified Clay

The compositions of the invention comprise a hydrophobically modified clay in an amount of from 0.01% to 5% by weight, preferably from 0.01% to 3% by weight, more preferably from 0.05% to 1% by weight based on the total weight of the composition. Higher levels of hydrophobically modified clays can give unpleasant tactile properties to the composition for some consumers.

Hydrophobically modified clays may be used in the present invention either singly or in combination with one or more other hydrophobically modified clays.

Suitable clays include hydrophobically modified natural clays and synthetic clays. In general, the term clay refers to a composition comprising particles which have a net electrostatic (ie, positive or negative charge) on at least one surface.

Preferably, the hydrophobically modified clay has a layered structure. In the compositions of the invention, the hydrophobically modified clay is advantageously present in the form of a dispersion or suspension of the clay particles.

Hydrophobically modified clays of the invention may be anionic or cationic, ie, they may have a net charge on the surface of the clay that is negative or positive, respectively. The term anionic clays and related terms, as used herein, refers to clays which are themselves anionic in nature ie, the clays themselves are negatively charged at their surface and are capable of exchanging cations. Similarly, the term cationic clays and related terms, as used herein, refers to clays which are themselves cationic in nature ie, the clays themselves are positively charged at their surface and are capable of exchanging anions.

Hydrophobically modified clays are derivable from clays by modification of the clay with a hydrophobic material.

Preferred anionic clays are clays from the smectite class of clays. Typically, clays of this type are crystalline, expandable, three-layer clays.

Smectite clays are, for example, disclosed in US Patents Nos 3,862,058, 3,948,790, 3,954,632 and 4,062,647 and in EP-A-299,575 and EP-A-313,146, all in the name of Procter & Gamble Company.

The term smectite clays herein includes both the clays in which aluminium oxide is present in a silicate lattice and the clays in which magnesium oxide is present in a silicate lattice. Typical smectite clay compounds include the compounds having the general formula Al₂(Si₂O₅)₂(OH)₂.nH₂O and the compounds having the general formula Mg₃(Si₂O₅)₂(OH)₂.nH₂O, and derivatives thereof, for example in which a proportion of the aluminium ions are replaced with magnesium ions or a proportion of the magnesium ions are replaced with lithium ions and/or some of the hydroxyl ions are replaced by fluoride ions; the derivatives may comprise a further metal ion to balance the overall charge. Smectite clays tend to adopt an expandable, three-layer structure.

The hydrophobically modified clay is preferably an expandable three-layer clay comprising at least 75% by weight of the clay of atoms selected from oxygen, silicon and aluminium and/or magnesium. More preferably, the hydrophobically modified clay comprises atoms selected from oxygen, silicon and aluminium and/or magnesium in an amount of at least 5% by weight of the clay, for each of the atoms.

Specific examples of suitable smectite clays include those selected from the classes of the montmorillonites, hectorites, volchonskoites, nontronites, saponites, beidelites and sauconites, particularly.those having an alkali or alkaline earth metal ion within the crystal lattice structure. Particularly preferred are hectorites, montmorillonites, nontronites, saponites, beidelites, sauconites and mixtures thereof. Preferred are montmorillonites, eg, bentonites and hectorites, with bentonites being particularly preferred.

The hydrophobically modified clay is preferably a hydrophobically treated bentonite clay.

It is customary to measure cation exchange capacity (sometimes termed "base exchange capacity") in terms of milliequivalents per 100g of clay (meq/100g). The cation exchange capacity of clays can be measured in several ways, including by electrodialysis, by exchange with ammonium ion followed by titration.or by a methylene blue procedure, all as fully set fourth in Grimshaw, "The Chemistry and Physics of Clays", pp. 264-265, Interscience (1971). The cation exchange capacity of a clay mineral relates to such factors as the expandable properties of the clay and the charge of the clay, which, in turn, is determined at least in part by the lattice structure, and the like.

Preferred anionic clays for use in the present invention have an ion exchange capacity of from 0.7meq/100g to 150meq/100g. Particularly preferred are clays having an ion exchange capacity of from 30 meq/100g to 100 meq/100g.

The clays preferably have an average particle size in the range of from 0.0001 µm to 800 µm, more preferably from 0.01 µm to 400 µm such as from 0.02 µm to 220 µm, even more preferably 0.02 µm to 100 µm. Particle sizes can be determined using a Malvern Mastersizer (Malvern Instruments, UK).

The hydrophobically modified clays used in the compositions of the invention preferably have organic ions replacing at least a proportion of inorganic metal ions by ion exchange processes known in the art. Preferably, the clay is hydrophobically modified by exchange into the clay of cations comprising one or more alkyl groups containing from 6 to 30 carbon atoms. The cationic group is preferably a quaternary ammonium group. Advantageously, the cations have the formula N⁺R¹R²R³R⁴ wherein R¹, R², R³ and R⁴ are independently (C₁ to C₃₀) alkyl, preferably (C₆ to C₃₀) alkyl, or benzyl. Preferably, one, two or three of R¹, R², R³ and R⁴ are independently (C₆ to C₃₀) alkyl and the other R¹, R², R³ and R⁴ group or groups are (C₁-C₆) alkyl or benzyl. Suitably, two of R¹, R², R³ and R⁴ are independently (C₆ to C₃₀) alkyl and the other R¹, R², R³ and R⁴ groups are (C₁-C₆) alkyl or benzyl. Optionally, the alkyl groups may comprise one or more ester (-OCO- or -COO-) and/or ether (-O-) linkages within the alkyl chain. Alkyl groups may be straight chain or branched and, for alkyl groups having 3 or more carbon atoms, cyclic. The alkyl groups may be saturated or may contain one or more carbon-carbon double bonds (eg, oleyl). The alkyl groups are optionally substituted with one or more hydroxyl groups. Alkyl groups are optionally ethoxylated with one or more ethyleneoxy groups on the alkyl chain. Preferably, the alkyl groups are straight chain, saturated groups.

Preferred compounds of formula N⁺R¹R²R³R⁴ which have two (C₆ to C₃₀) alkyl groups include: cetyl, stearyl an behenyl trimethylammonium chloride.

Preferred compounds of formula N⁺R¹R²R³R⁴ which have two (C₆ to C₃₀) alkyl groups include:
Distearyldimethylammonium chloride (distearyl dimonium chloride);
Distearyldimethylammonium bromide (distearyl dimonium bromide);
Dicetyldimethylammonium chloride (dicetyl dimonium chloride);
Dicetyldimethylammonium bromide (dicetyl dimonium bromide); Dimethyldi(hydrogenated tallow)ammonium chloride (Quaternium-18);
Dicetylmethylbenzylammonium chloride;
Dicocodimethylammonium chloride (dicoco dimonium chloride);
Dicocodimethylammonium bromide (dicoco dimonium bromide);
Dibehenyl/diarachidyldimethylammonium bromide (dibehenyl/diarachidyl dimonium bromide);
Dibehenyl/diarachidyldimethylammonium chloride (dibehenyl/diarachidyl dimonium chloride);
Dibehenyl dimonium methyl sulfate (dibehenyl dimonium methyl sulfate);
Hydroxypropyl bis-stearylammonium chloride (hydroxypropyl bis-stearyl dimonium chloride);
Dibehenyldimethylammonium chloride (dibehenyl dimonium chloride);
Dibehenylmethylbenzylammonium chloride;
Dimyristyldimethylammonium chloride (dimyristyl dimonium chloride); and
Dimyristyldimethylammonium bromide (dimyristyl dimonium bromide).

Preferred compounds of formula N⁺R¹R²R³R⁴ which have three (C₆ to C₃₀) alkyl groups include compounds which have three alkyl groups having 8 to 22 carbon atoms and one alkyl group having 1 to 4 carbon atoms, such as, for example:
Tricetylmethylammonium chloride;
Tricetylmethylammonium bromide;
Tricetylmethylammonium methylsulfate;
Tri((C₈-C₁₀)alkyl)methylammonium chloride;
Tri((C₈-C₁₀)alkyl)methylammonium bromide; and
Tri((C₈-C₁₀)alkyl)methylammonium methylsulfate.

A particularly preferred material is bentonite modified with Quaternium-18 (ie, di-hydrogenated tallow dimethyl ammonium cations). An example of such a product is Tixogel MP 100™ from Sud Chemie. Other suitable hydrophobically modified clays include Quaternium benzalkonium bentonite, Quaternium-18 hectorite, stearalkonium bentonite, stearalkonium hectorite and dihydrogenated tallow benzylmonium hectorite.

### Hair Fibre Modifying Agent

The compositions of the invention further comprise one or more hair fibre modifying agents that are capable of reducing the stiffness of a hair fibre. Hair fibre modifying agents have been found to give particular advantages in terms of the hair conditioning properties of the compositions when used in combination with hydrophobically modified clays according to the invention. Hair fibre modifying agents can be identified by the skilled person by measuring the change in stiffness of hair fibres using the method described herein in the examples section below.

Hair fibre modifying agents for use in the invention are preferably capable of reducing the stiffness of a hair fibre by at least 5%, more preferably at least 10%, even more preferably at least 15% relative to the stiffness of the original (ie, untreated) hair fibre.

Hair fibre modifying agents can be insoluble in water at 25°C (ie, having a solubility of less than 1 gram per litre, preferably less than 0.1 gram per litre in water). Such hair fibre modifying agents will typically be liquid at 25°C. Examples of insoluble hair fibre modifying agents include the following (u denotes unified atomic mass units):
- mineral oils, preferably those having a low viscosity and/or low molecular weight, typically a molecular weight below 600 u (eg, from 120 to 600 u) and/or a viscosity below 4cSt (mm²sec⁻¹) as measured using a Brookfield viscometer at 18°C using an LV1 spindle; for example, oils comprising straight chain or branched chain, saturated or unsaturated hydrocarbons having from 10 to 44 carbon atoms, optionally comprising one or more phenyl groups;
- functionalised oils, preferably compounds containing one or more groups selected from ether, ester, keto, aldehyde, carboxyl, alcohol, diol, polyol, amino, amido, thiol, thioether, and preferably containing 8 to 44 carbon atoms; specific examples are isoamyl ether, isopropyl myristate, octan-2-one, decyl alcohol and 1,10-decanediol;
- saccharide polyesters (eg, esters of sucrose with carboxylic acids having from 4 to 36 carbon atoms, such as sucrose pentaerucate);
- silicone oils, preferably low molecular weight (eg, having a molecular weight of below 2000 u such as from 300 to 2000 u) straight chain dimethicones or cyclomethicones; a suitable commercial product is DC 245 from Dow Corning;
- triglyceride oils, preferably those containing acyl groups comprising from 6 to 24 carbon atoms (including the carbon atom of the C=O group).

A particularly preferred hair fibre modifying agent of the type mentioned above is a hydrocarbon oil. The hydrocarbon oils may comprise straight chain or branched alkanes having from 8 to 16 carbon atoms. Most preferred hydrocarbon oils are light mineral oils, such as paraffin oils eg, C11-C13 isoparaffin oils. An example of a commercial product is Isopar L ™ which is available from Exxon.

Insoluble hair fibre modifying agents are typically hydrophobic. By the term hydrophobic in relation to the hair fibre modifying agents, it is meant that the agents have a greater solubility in octan-1-ol than water at 25°C. Preferably, the agents have a log P value (wherein P is the n-octanol/water partition coefficient and log P means log₁₀ P) of greater than 1, preferably greater than 2, more preferably greater than 3. Log P values can be determined as described in J Sangster, Octanol-water partition coefficients of simple organic compounds, J Phys Chem Ref Data, 18, 1111, 1989, the contents of which are incorporated by reference herein, and are typically determined at 25°C. Preferably, log P values are measured at 25°C.

Alternatively, hair fibre modifying agents for use in the invention can be soluble in water at 25°C (ie, having a solubility of at least 1 gram per litre in water). Examples of soluble hair fibre modifying agents include the following:
- amino acids (such as any of the twenty naturally occurring amino acids in optically active (eg, enantiomeric) or racemic forms), for example arginine and lysine;
- urea;
- methyl amines or their N-oxides, for example betaine and trimethylamine oxide;
- mono-, di- or poly-carboxylic acids, preferably having from 1 to 12 carbon atoms and optionally comprising one or more hydroxyl groups, such as citric acid and lactic acid;
- carbohydrates, such as mono- or di-saccharides eg, glucose and sucrose; and
- polyols, such as sorbitol, mannitol and glycerol.

Urea is a particularly preferred water-soluble hair fibre modifying agent.

The one or more hair fibre modifying agents are present in the composition in an amount of from 0.001% to 10% by weight, more preferably 0.01% to 5% by weight, most preferably 0.01% to 2% by weight.

### Cationic Surfactant

Compositions in accordance with the invention are formulated as conditioners for the treatment of hair (typically after shampooing) and subsequent rinsing.

The compositions comprise one or more conditioning surfactants which are cosmetically acceptable and suitable for topical application to the hair.

Suitable conditioning surfactants include those selected from cationic surfactants, used singly or in admixture. Examples include quaternary ammonium hydroxides or salts thereof, eg, chlorides, such as of the type mentioned hereinbefore. Preferably, the cationic surfactants have the formula N⁺R^{1a}R^{2a}R^{3a}R^{4a} wherein R^{1a}, R^{2a}, R^{3a} and R^{4a} are independently (C₁ to C₃₀) alkyl or benzyl. Preferably, one, two or three of R^{1a}, R^{2a}, R^{3a} and R^{4a} are independently (C₄ to C₃₀) alkyl and the other R^{1a}, R^{2a}, R^{3a} and R^{4a} group or groups are (C₁-C₆) alkyl or benzyl. More preferably, one or two of R^{1a}, R^{2a}, R^{3a} and R^{4a} are independently (C₆ to C₃₀) alkyl and the other R^{1a}, R^{2a}, R^{3a} and R^{4a} groups are (C₁-C₆) alkyl or benzyl groups. Optionally, the alkyl groups may comprise one or more ester (-OCO- or -COO-) and/or ether (-O-) linkages within the alkyl chain. Alkyl groups may optionally be substituted with one or more hydroxyl groups. Alkyl groups may be straight chain or branched and, for alkyl groups having 3 or more carbon atoms, cyclic. The alkyl groups may be saturated or may contain one or more carbon-carbon double bonds (eg, oleyl). Alkyl groups are optionally ethoxylated on the alkyl chain with one or more ethyleneoxy groups.

Suitable cationic surfactants for use in hair conditioners of the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, dihydrogenated tallow dimethyl ammonium chloride (eg, Arquad 2HT/75 from Akzo Nobel), cocotrimethylammonium chloride, PEG-2-oleammonium chloride and the corresponding hydroxides thereof. Further suitable cationic surfactants include those materials having the CTFA designations Quaternium-5, Quaternium-31 and Quaternium-18. Mixtures of any of the foregoing materials may also be suitable. A particularly useful cationic surfactant for use in hair conditioners of the invention is cetyltrimethylammonium chloride, available commercially, for example as GENAMIN CTAC, ex Hoechst Celanese. Another particularly useful cationic surfactant for use in hair conditioners of the invention is behenyltrimethylammonium chloride, available commercially, for example as GENAMIN KDMP, ex Clariant.

In particular, it is preferred if a combination of at least two cationic surfactants is used, where one surfactant is a C₁₆-C₂₂ alkyl trimethylammonium salt, and the second surfactant has the formula N⁺R^{1a}R^{2a}R^{3a}R^{4a} wherein of R^{1a}, R^{2a}, R^{3a} and R^{4a} are independently (C₁₆ to C₂₂) alkyl and the other R^{1a}, R^{2a}, R^{3a} and R^{4a} group or groups are (C₁-C₆) hydrocarbyl.

Another example of a class of suitable cationic surfactants for use in the invention, either alone or together with one or more other cationic surfactants, is a combination of (i) and (ii) below:
(i) an amidoamine corresponding to the general formula (I): 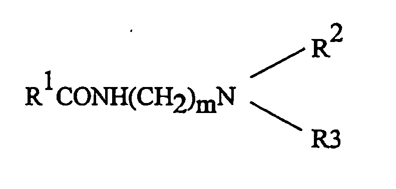 in which R¹ is a hydrocarbyl chain having 10 or more carbon atoms,
   R² and R³ are independently selected from hydrocarbyl chains of from 1 to 10 carbon atoms, and
   m is an integer from 1 to about 10; and
(ii) an acid.

As used herein, the term hydrocarbyl chain means an alkyl or alkenyl chain.

Preferred amidoamine compounds are those corresponding to formula (I) in which
R¹ is a hydrocarbyl residue having from about 11 to about 24 carbon atoms,
R² and R³ are each independently hydrocarbyl residues, preferably alkyl groups, having from 1 to about 4 carbon atoms, and
m is an integer from 1 to about 4.

Preferably, R² and R³ are methyl or ethyl groups.

Preferably, m is 2 or 3, i.e. an ethylene or propylene group.

Preferred amidoamines useful herein include stearamidopropyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behenamidopropyldimethylamine, behenamidopropyldiethylmine, behenamidoethyldiethylamine, behenamidoethyldimethylamine, arachidamidopropyldimethylamine, arachidamidopropyldiethylamine, arachidamidoethyldiethylamine, arachidamidoethyldimethylamine, and mixtures thereof.

Particularly preferred amidoamines useful herein are stearamidopropyldimethylamine, stearamidoethyldiethylamine, and mixtures thereof.

Commercially available amidoamines useful herein include: stearamidopropyldimethylamine with tradenames LEXAMINE S-13 available from Inolex (Philadelphia Pennsylvania, USA) and AMIDOAMINE MSP available from Nikko (Tokyo, Japan), stearamidoethyldiethylamine with a tradename AMIDOAMINE S available from Nikko, behenamidopropyldimethylamine with a tradename INCROMINE BB available from Croda (North Humberside, England), and various amidoamines with tradenames SCHERCODINE series available from Scher (Clifton New Jersey, USA).

In compositions of the invention, the level of amidoamine (i) is preferably from 0.01 to 10%, more preferably 0.1 to 7.5%, yet more preferably 0.2 to 6%, and most preferably from 0.2 to 5% by weight based on total weight of the composition.

Acid (ii) may be any organic or mineral acid which is capable of protonating the amidoamine in the hair treatment composition. Suitable acids useful herein include hydrochloric acid, acetic acid, tartaric acid, fumaric acid, lactic acid, malic acid, succinic acid, and mixtures thereof. Preferably, the acid is selected from the group consisting of acetic acid, tartaric acid, hydrochloric acid, fumaric acid, and mixtures thereof.

The primary role of the acid is to protonate the amidoamine in the hair treatment composition thus forming a tertiary amine salt (TAS) in situ in the hair treatment composition. The TAS in effect is a non-permanent quaternary ammonium or pseudo-quaternary ammonium cationic surfactant.

Suitably, the acid is included in a sufficient amount to protonate all the amidoamine present, i.e. at a level which is at least equimolar to the amount of amidoamine present in the composition.

Suitably, the acid is included in a sufficient amount to provide the hair treatment composition with a pH of between from about 2.5 to about 7.0, preferably from about 4 to about 6.5.

Typically, when an acid is present, the hair treatment compositions of the present invention comprise from about 0.1% to about 10.0% and preferably from about 0.2% to about 5.0 % by weight of a suitable acid.

In conditioners of the invention, the level of cationic surfactant is preferably from 0.01 to 10%, more preferably 0.05 to 5%, most preferably 0.1 to 3% by weight of the composition.

### Fatty Alcohol

Conditioners of the invention also incorporate a fatty alcohol. The combined use of fatty alcohols and cationic surfactants in conditioning compositions is believed to be especially advantageous, because this leads to the formation of a lamellar phase, in which the cationic surfactant is dispersed.

Suitable fatty alcohols comprise from 8 to 22 carbon atoms, more preferably 16 to 22. Fatty alcohols are typically compounds containing straight chain alkyl groups. Examples of suitable fatty alcohols include cetyl alcohol, stearyl alcohol and mixtures thereof. The use of these materials is also advantageous in that they contribute to the overall conditioning properties of compositions of the invention.

The level of fatty alcohol in conditioners of the invention is conveniently from 0.01 to 10%, preferably from 0.1 to 8%, more preferably from 0.2 to 7%, most preferably from 0.3 to 6% by weight of the composition. The weight ratio of cationic surfactant to fatty alcohol is suitably from 1:1 to 1:10, preferably from 1:1.5 to 1:8, optimally from 1:2 to 1:5. If the weight ratio of cationic surfactant to fatty alcohol is too high, this can lead to eye irritancy from the composition. If it is too low, it can make the hair feel squeaky for some consumers.

### Block Copolymer

Compositions of the invention also comprise a block copolymer according to the formula 1 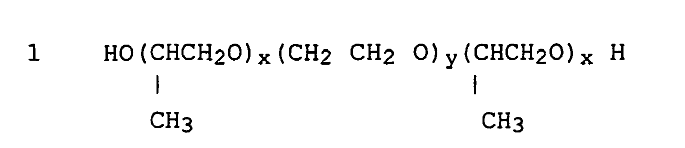 wherein the mean value of y is from 2 to 30 and the mean value x is from 12 to 30 and further the value of x/y is from 1 to 6.

This is a block copolymer based on polyethyleneoxide (EO) and polypropyleneoxide (PO) blocks.

The mean molecular weight is suitably measured by determining the hydroxyl number for the polymer then transforming this into molecular weight. This corresponds to a number based mean molecular weight.

Suitable block copolymers for incorporation into compositions according to the invention are supplied by BASF corporation under the trade name Pluronic ^{(RTM)} R. They have the INCI name Meroxapol.

In formula 1, the degree of polymerisation, x, is indicated as the same for each polypropyleneoxide block. For the sake of clarity, it should be explained that these degrees of polymerisation are mean values and are approximately the same rather than identical for any particular formula.

This is a result of the polymerisation methods used for production of the compounds.

Suitably, the level of block copolymer is from 0.01% to 5% by weight of the composition, preferably from 0.05% to 2%, more preferably from 0.07% to 0.5%.

The mean value of y in formula 1 should be from 2 to 30, preferably from 3 to 20, more preferably from 4 to 15. The mean value of x in formula 1 should be from 12 to 30, preferably from 15 to 28.

Further to the constrains on the values of x and y in formula 1, it is also necessary for the value of x divided by y (x/y) to be from 1 to 6, more preferably from 2 to 4.

### Conditioning Agents

The compositions of the invention may also contain one or more further conditioning agents. As used herein, the term "conditioning agent" includes any material which is used to give a particular conditioning benefit to hair and/or skin. For example, suitable materials are those which deliver one or more benefits relating to shine, softness, combability, wet-handling, anti-static properties, protection against damage, body, volume, stylability and manageability.

Preferred conditioning agents for use in the present invention include emulsified silicones, used to impart for example wet and dry conditioning benefits to hair such as softness, smooth feel and ease of combability.

Various methods of making emulsions of particles of silicones for use in the invention are available and are well known and documented in the art.

The viscosity of the silicone itself (not the emulsion or the final washing composition) preferably ranges from 10,000 cSt (mm² sec⁻¹) to 5 million cSt (mm² sec⁻¹) at 25°C. The viscosity can be measured by means of a glass capillary viscometer as set out further in Dow Corning Corporate Test Method CTM004 July 20 1970. For high viscosity silicones, dynamic viscosity can be measured using a constant shear rheometer at a low shear rate of 1 sec⁻¹ and the kinematic viscosity calculated using the density of the silicone.

Suitable silicones include polydiorganosiloxanes, in particular polydimethylsiloxanes which have the CTFA designation dimethicone. An example is dimethicone fluid having a viscosity of up to 100,000 centistokes (mm² sec⁻¹) at 25° C, which is available commercially from the General Electric Company as the Viscasil series and from Dow Corning as the DC 200 series.

Aminofunctional silicones which have the CTFA designation amodimethicone, are also suitable for use in the compositions of the invention, as are polydimethyl siloxanes having hydroxyl end groups, which have the CTFA designation dimethiconol.

Also suitable are silicone gums. "Silicone gum" denotes polydiorganosiloxanes having a molecular weight of from 200,000 to 1,000,000 u and specific examples include dimethicone gums, dimethiconol gums, polydimethyl siloxane/diphenyl/methylvinylsiloxane copolymers, polydimethylsiloxane/methylvinylsiloxane copolymers and mixtures thereof. Examples include those materials described in US Pat. No. 4,152,416 (Spitzer), and on General Electric Silicone Rubber product Data Sheet SE 30, SE 33, SE 54 and SE 76.

Also suitable for use in the present invention are silicone gums having a slight degree of cross-linking, as are described for example in WO 96/31188. These materials can impart body, volume and stylability to hair, as well as good wet and dry conditioning.

Preferred emulsified silicones for use in compositions of the invention have an average silicone particle diameter (D_{3,2}) in the composition of less than 100, preferably less than 30, more preferably less than 20 microns, most preferably less than 10 microns.
Particle size may be measured by means of a laser light scattering technique, using a 2600D Particle Sizer from Malvern Instruments.

Suitable silicone emulsions for use in the invention are commercially available in a pre-emulsified form. This is particularly preferred since the pre-formed emulsion can be incorporated into the washing composition by simple mixing.

Examples of suitable pre-formed emulsions include emulsions DC2-1766 and DC2-1784, and DC 1785 available from Dow Corning. These are emulsions of dimethiconol. Cross-linked silicone gums are also available in a pre-emulsified form, which is advantageous for ease of formulation. A preferred example is the material available from Dow Corning as DC X2-1787, which is an emulsion of cross-linked dimethiconol gum.

The amount of silicone conditioning agent incorporated into the compositions of the invention depends on the level of conditioning desired and the material used. A preferred amount is from 0.01 to about 5% by weight of the total composition although these limits are not absolute. The lower limit is determined by the minimum level to achieve conditioning and the upper limit by the maximum level to avoid making the hair and/or skin unacceptably greasy. We have found that an amount of silicone of from 0.5 to 4% by weight of the total composition, is a particularly suitable level.

A further preferred class of conditioning agents are peralk(en)yl hydrocarbon materials, used to enhance the body, volume and stylability of hair.

EP 567 326 and EP 498 119 describe suitable peralk(en)yl hydrocarbon materials for imparting stylability and enhanced body to hair. Preferred materials are polyisobutylene materials available from Presperse, Inc. under the PERMETHYL trade name.

The amount of per-alk(en)yl hydrocarbon material incorporated into the compositions of the invention depends on the level of body and volume enhancement desired and the specific material used. A preferred amount is from 0.01 to about 10% by weight of the total composition although these limits are not absolute. The lower limit is determined by the minimum level to achieve the body and volume enhancing effect and the upper limit by the maximum level to avoid making the hair unacceptably stiff. We have found that an amount of per-alk(en)yl hydrocarbon material of from 0.5 to 2% by weight of the total composition is a particularly suitable level.

### Water

Compositions of the invention also comprise water.
Typically, water is present in an amount of from 10% to 95% by weight of the composition, preferably, from 25% to 95% by weight, more preferably 30% to 95% by weight, most preferably 35% to 95% by weight.

### Viscosity Enhancer

Compositions of the invention may also optionally include a viscosity enhancer.
Examples of viscosity enhancers include:
cellulose derivatives such as methylcellulose, hydroxymethylcellulose, hydroxyethyl cellulose, hydroxypropylcellulose, and hydroxypropyl methylcellulose;
water-soluble salts of cellulose ethers such as sodium carboxymethyl cellulose and sodium carboxymethyl hydroxyethyl cellulose;
natural gums such as carrageenan, xanthan gum, gum arabic, gum tragacanth and guar gum and derivatives thereof such as hydroxypropyl guar and guar hydroxypropyl trimonium chloride;
inorganic thickeners such as colloidal magnesium aluminium silicate (Veegum), finely divided silica, natural clays such as bentonite and synthetic clays such as the synthetic hectorite available as Laponite(ex Laporte Industries Ltd);
vinyl-type polymeric thickeners such as polyvinylpyrrolidone, polyvinyl alcohol, sodium acrylate/vinyl alcohol copolymers and carboxyvinyl polymers, such as those polymers of acrylic acid cross-linked with about 0.75% to 2.0% of polyallylsucrose or polyallylpentaerythritol, obtainable under the Carbopol trademark from B.F.Goodrich.

As the viscosity enhancer, cellulose derivatives are particularly preferred, especially hydroxyethyl cellulose.

### Other Ingredients

Compositions of this invention may optionally contain any other ingredient normally used in hair treatment formulations. These other ingredients may include viscosity modifiers, preservatives, colouring agents, polyols such as glycerine and polypropylene glycol, chelating agents such as EDTA, antioxidants, fragrances, and sunscreens. It is also possible to include conventional poloxamers in the compositions of the invention, preferably low HLB poloxamers with an HLB of 10 or less. Each of these ingredients will be present in an amount effective to accomplish its purpose. Generally these optional ingredients are included individually at a level of up to about 5% by weight of the total composition.

### Use

The method of treating hair according to the invention comprises applying to the hair a composition of the invention. The method preferably involves the application of the composition to wet hair, typically after it has been washed and rinsed. Preferably, after treatment with the composition, the treated hair is rinsed with water and dried at a temperature above room temperature or allowed to dry at room temperature.

The invention will now be illustrated with reference to the following non-limiting examples. In the examples and throughout this specification all percentages are percentages by weight of the total composition unless indicated otherwise.

### Examples

Compositions were prepared according to the details in Table 1

**Table 1**

| **Chemical Name** | **Trade Name** | **A** | **1** |
|---|---|---|---|
| Cetrimonium Chloride | Genamin CTAC | 1.68 | 1.68 |
| Di-hydrogenated tallow dimethyl ammonium chloride | Arquad 2HT-75 | 0.8 | 0.8 |
| Cetearyl alcohol | | 5 | 5 |
| Quaternium 18 bentonite | Tixogel MP100 | 0.1 | 0.1 |
| C11-C13 isoparaffin | Isopar L | 0.5 | 0.5 |
| block copolymer | Pluronic 31R1 | - | 0.1 |
| amodimethicone/ dimethicone silicone emulsion | PCP 2090 1 micrometre droplets | 3 | 3 |
| prefume, minors, water | | to 100 | to 100 |

All figures in table 1 are expressed as weight percent of the total composition for the active ingredient. The pluronic 31R1 is according to formula 1 and has x=27 and y=8 (approximately). x/y is 3.4.

Example A is a comparative example while example 1 is according to the invention.

The compositions were compared in a half-head test, where subjects had half their head washed then conditioned with A and the other half washed and conditioned with 1. The data in table 2 represent the numbers of panellists preferring either A or 1 for the attribute listed for the hair once dried.

The results show the composition according to the invention is highly preferred for attributes linked to the smoothness and slipperiness of the hair.

**Table 2**

| **Attribute** | **Preferring A** | **Preferring 1** |
|---|---|---|
| Overall like | 17 | 26 |
| Powdery feel | 19 | 17 |
| Slippery feel | 18 | 24 |
| Smooth feel | 17 | 23 |
| Moisturised feel | 13 | 25 |

## Claims

1. A hair conditioning composition with a pH of 8 or less comprising:
(a) 0.01% to 10% by weight of one or more cationic surfactants;
(b) 0.01% to 10% by weight of one or more fatty alcohols having from 8 to 22 carbon atoms;
(c) 0.001% to 5% by weight of a hydrophobically modified clay; and
(d) from 10% to 95% by weight of water, and
(e) from 0.001% to 10% by weight of a hair fibre modifying agent that is capable of reducing the stiffness of a hair fibre,
wherein the weight ratio of cationic surfactant to fatty alcohol is from 1:1 to 1:10, **characterised in that** the composition further comprises from 0.01% to 5% by weight of a block copolymer according to the formula 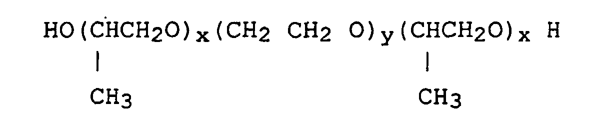 wherein the mean value of y is from 2 to 30 and the mean value x is from 12 to 30 and further the value of x/y is from 1 to 6.

2. A composition according to claim 1, wherein the hair fibre modifying agent is selected from: mineral oils; functionalised oils comprising one or more groups selected from ether, ester, keto, aldehyde, carboxyl, alcohol, diol, polyol, amino, amido, thiol and thioether, and containing 8 to 44 carbon atoms; saccharide polyesters; silicone oils; triglyceride oils and mixtures thereof.

3. A composition according to claim 2, wherein the hair fibre modifying agent is a hydrocarbon oil.

4. A composition according to claim 2 wherein the hair fibre modifying agent is isopropyl myristate.

5. A composition according to Claim 1 wherein the hair fibre modifying agent is selected from: amino acids; urea; methyl amines and their N-oxides; mono-, di- or poly-carboxylic acids; carbohydrates; polyols and mixtures thereof.

6. A composition as claimed in Claim 5, wherein the hair fibre modifying agent is urea.

7. A composition according to any preceding claim, wherein the hydrophobically modified clay is an expandable three-layer clay comprising at least 75% by weight of the clay of atoms selected from oxygen, silicon, aluminum, magnesium and mixtures thereof.

8. A composition as claimed in any one of Claims 1 to 7, wherein the hydrophobically modified clay is a hydrophobically modified bentonite clay.

9. A composition as claimed in any one of Claims 1 to 8, wherein the clay is hydrophobically modified by exchange into the clay of cations comprising at least one hydrocarbyl group comprising from 6 to 30 carbon atoms.

10. A composition as claimed in Claim 9, wherein the cations have the formula N⁺R¹R²R³R⁴ wherein R¹, R², R³ and R⁴ are independently (C₁ to C₃₀) hydrocarbyl groups.

11. A composition as claimed in any one of Claims 1 to 10 which further comprises from 0.01% to 5% by weight of a silicone conditioning agent.

12. A method of treating hair which comprises applying to the hair a composition of any one of Claims 1 to 11.

13. The use of compositions according to any one of claims 1 to 10 for the conditioning of hair.

## Patentansprüche

1. Haar konditionierende Zusammensetzung mit einem pH-Wert von 8 oder weniger, umfassend:
(a) 0,01 % bis 10 Gewichtsprozent von einem oder mehreren kationischen Tensiden;
(b) 0,01 % bis 10 Gewichtsprozent von einem oder mehreren Fettalkoholen mit 8 bis 22 Kohlenstoffatomen;
(c) 0,001 % bis 5 Gewichtsprozent von einem hydrophob modifizierten Ton; und
(d) 10 % bis 95 Gewichtsprozent Wasser; und
(e) 0,001 % bis 10 Gewichtsprozent eines Haarfaser modifizierenden Mittels, das die Steifigkeit einer Haarfaser vermindern kann,
wobei das Gewichtsverhältnis von kationischem Tensid zu Fettalkohol 1 : 1 bis 1 : 10 ist, **dadurch gekennzeichnet, dass** die Zusammensetzung weiterhin 0,01 % bis 5 Gewichtsprozent von einem Blockcopolymer gemäß der Formel 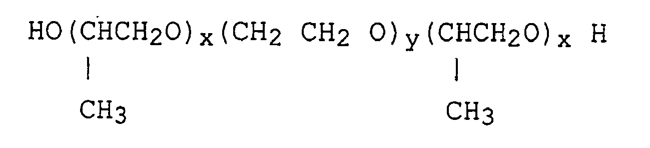 worin der Mittelwert von y 2 bis 30 ist und der Mittelwert von x 12 bis 30 ist und weiterhin der Wert von x/y 1 bis 6 ist, umfasst.

2. Zusammensetzung nach Anspruch 1, worin das Haarfaser modifizierende Mittel ausgewählt ist aus:
Mineralölen, funktionalisierten Ölen, umfassend eine oder mehrere Gruppen, ausgewählt aus Ether, Ester, Keto, Aldehyd, Carboxyl, Alkohol, Diol, Polyol, Amino, Amido, Thiol und Thioether und enthaltend 8 bis 44 Kohlenstoffatome; Saccharidpolyestern; Silikonölen; Triglyceridölen und Gemischen davon.

3. Zusammensetzung nach Anspruch 2, worin das Haarfaser modifizierende Mittel ein Kohlenwasserstofföl ist.

4. Zusammensetzung nach Anspruch 2, worin das Haarfaser modifizierende Mittel Myristinsäureisopropylester ist.

5. Zusammensetzung nach Anspruch 1, worin das Haarfaser modifizierende Mittel ausgewählt ist aus: Aminosäuren; Harnstoff; Methylaminen und deren N-Oxiden; Mono-, Di- oder Polycarbonsäuren; Kohlenhydraten; Polyolen und Gemischen davon.

6. Zusammensetzung nach Anspruch 5, worin das Haarfaser modifizierende Mittel Harnstoff ist.

7. Zusammensetzung nach einem vorangehenden Anspruch, worin der hydrophob modifizierte Ton ein blähfähiger Dreischichtton, umfassend mindestens 75 Gewichtsprozent des Tons von Atomen, ausgewählt aus Sauerstoff, Silizium, Aluminium, Magnesium und Gemischen davon, ist.

8. Zusammensetzung nach einem der Ansprüche 1 bis 7, worin der hydrophob modifizierte Ton ein hydrophob modifizierter Bentonitton ist.

9. Zusammensetzung nach einem der Ansprüche 1 bis 8, worin der Ton durch Austausch von Kationen, umfassend mindestens eine Kohlenwasserstoffgruppe, umfassend 6 bis 30 Kohlenstoffatome, in den Ton hydrophob modifiziert ist.

10. Zusammensetzung nach Anspruch 9, worin die Kationen die Formel N⁺R¹R²R³R⁴ aufweisen, worin R¹, R², R³ und R⁴ unabhängig (C₁ bis C₃₀) Kohlenwasserstoffgruppen darstellen.

11. Zusammensetzung nach einem der Ansprüche 1 bis 10, die weiterhin 0,01 % bis 5 Gewichtsprozent eines Silikonkonditionierungsmittels umfasst.

12. Verfahren zum Behandeln von Haar, das Auftragen einer Zusammensetzung nach einem der Ansprüche 1 bis 11 auf das Haar umfasst.

13. Verwendung der Zusammensetzungen nach einem der Ansprüche 1 bis 10 für das Konditionieren von Haar.

## Revendications

1. Composition après-shampooing avec un pH de 8 ou inférieur comprenant .
(a) 0,01 % à 10 % en poids d'un ou plusieurs tensioactifs cationiques ;
(b) 0,01 % à 10 % en poids d'un ou plusieurs alcools gras ayant de 8 à 22 atomes de carbone ;
(c) 0,001 % à 5 % en poids d'une argile hydrophobiquement modifiée ; et
(d) de 10 % à 95 % en poids d'eau, et
(e) de 0,001% à 10 % en poids d'un agent de modification de la fibre capillaire qui est capable de réduire la dureté d'une fibre capillaire,
dans laquelle le rapport massique du tensioactif cationique à l'alcool gras est de 1:1 à 1:10, **caractérisée en ce que** la composition comprend en outre de 0,01 % à 5 % en poids d'un copolymère séquencé selon la formule 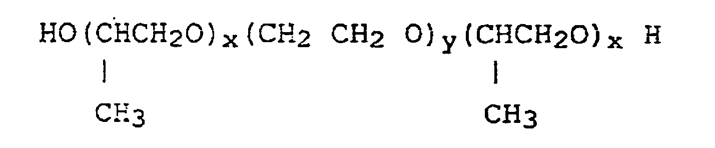 dans laquelle la valeur moyenne de y est de 2 à 30 et la valeur moyenne de x est de 12 à 30 et en outre la valeur de x/y est de 1 à 6.

2. Composition selon la revendication 1, dans laquelle l'agent de modification de la fibre capillaire est choisi parmi: des huiles minérales ; des huiles fonctionnalisées comprenant un ou plusieurs groupes choisis parmi l'éther, l'ester, la cétone, l'aldéhyde, le carboxyle, l'alcool, le diol, le polyol, l'amino, l'amido, le thiol et le thioéther, et contenant de 8 à 44 atomes de carbone ; des polyesters de saccharide ; des huiles de silicone ; des huiles de triglycéride et des mélanges de ceux-ci.

3. composition selon la revendication 2, dans laquelle l'agent de modification de la fibre capillaire est une huile d'hydrocarbures.

4. Composition selon la revendication 2, dans laquelle l'agent de modification de la fibre capillaire est l'isopropyl myristate.

5. Composition selon la revendication 1, dans laquelle l'agent de modification de la fibre capillaire est choisi parmi : des acides aminés ; l'urée ; des méthylamines et leurs N-oxydes ; des acides mono-, di- ou polycarboxyliques ; des hydrates de carbone ; des polyols et des mélanges de ceux-ci.

6. Composition selon la revendication 5, dans laquelle l'agent de modification de la fibre capillaire est l'urée.

7. Composition selon l'une quelconque des revendications précédentes, dans laquelle l'argile hydrophobiquement modifiée est une argile à trois couches extensible comprenant au moins 75 % en poids de l'argile d'atomes choisis parmi l'oxygène, le silicium, l'aluminium, le magnésium et des mélanges de ceux-ci.

8. Composition selon l'une quelconque des revendications 1 à 7, dans laquelle l'argile hydrophobiquement modifiée est une argile bentonite hydrophobiquement modifiée.

9. Composition selon l'une quelconque des revendications 1 à 8, dans laquelle l'argile est hydrophobiquement modifiée par échange dans l'argile de cations comprenant au moins un groupe hydrocarbyle comprenant de 6 à 30 atomes de carbone.

10. Composition selon la revendication 9, dans laquelle les cations ont pour formule N⁺R¹R²R³R⁴ dans laquelle R¹, R² R³ et R⁴ indépendamment sont des groupes hydrocarbyle (C₁ à C₃₀) .

11. Composition selon l'une quelconque des revendications 1 à 10 qui comprend en outre de 0,01 % à 5 % en poids d'un agent de conditionnement de silicone.

12. Méthode de traitement des cheveux qui comprend l'application sur les cheveux d'une composition selon l'une quelconque des revendications 1 à 11.

13. Utilisation de compositions selon l'une quelconque des revendications 1 à 10 pour le conditionnement des cheveux.
